# EUROPEAN PATENT SPECIFICATION

(11) **EP 0 514 936 B1**
(45) Date of publication and mention of the grant of the patent: **27.12.1996**
(21) Application number: 92108704.5
(22) Date of filing: 22.05.1992
(51) Int. Cl.: C07K 5/075, C07K 1/00, A23L 1/236

(54) **Method of preparing alpha-L-aspartyl-L-phenylalanine methyl ester**
Verfahren zur Herstellung von Alpha-Aspartyl-Phenylalanin-Methylester
Procédé de préparation de l'ester-méthylique de l'alpha-aspartyl phénylalanine

(30) Priority: 23.05.1991 JP 221334/91
(43) Date of publication of application: 25.11.1992
(73) Proprietor: Ajinomoto Co., Inc., Tokyo 104 (JP)
(72) Inventor: Kishimoto, Shinichi, c/o Central Res.Lab., Kawasaki-shi, Kanagawa-ken (JP); Sakamoto, Kimiyasu, c/o Central Res. Lab., Kawasaki-shi, Kanagawa-ken (JP); Abe, So, c/o Central Research Lab., Kawasaki-shi, Kanagawa-ken (JP); Kato, Toshihisa, c/o Central Res. Lab., Kawasaki-shi, Kanagawa-ken (JP)
(74) Representative: Strehl Schübel-Hopf Groening & Partner

(56) References cited:
- EP-A- 0 099 960
- WO-A-83/01619
- CHEMICAL ABSTRACTS, vol. 79, no. 11, September 17, 1973, Columbus, Ohio, USA ARIYOSHI YASUO et al. "Synthesis of a sweet peptide, alpha-L-aspartyl-L- - phenylalanine methyl ester, without the use of protecting groups" page 484, column 2, abstract-no. 66 803b
- CHEMICAL ABSTRACTS, vol. 85, no. 1, July 5, 1976, Columbus, Ohio, USA UCHIYAMA
- NOBORU et al. "Alpha-L-Aspartyl-L-phenyl- alanine lower alkyl esters" page 492, column 1, abstract-no. 6 056z
- CHEMICAL ABSTRACTS, vol. 87, no. 17, October 24, 1977, Columbus, Ohio, USA FUJINO MASAHIKO et al. "Aspartyl dipeptide esters" page 800, column 1, abstract-no. 136 391g

## Description

The present invention relates to a method of preparing α-L-aspartyl-L-phenylalanine methyl ester (hereinafter referred to as "α-APM") which is useful as a sweetener, specifically it relates to an extremely improved method of preparing α -APM by neutralizing an acid addition salt of α -APM with a base.

More precisely, it relates to a method of neutralizing an acid addition salt of α -APM in an aqueous medium, in which the neutralization is effected under particular concentration and particular temperature conditions and is followed by cooling to cause crystallization of α -APM.

α -APM of the present invention is a dipeptide sweetener having a sweetness of about 200 times that of sucrose (cane sugar). Because of the extremely good sweetening properties and the low caloric value, it has become used significantly as a diet sweetener in these days, and the worldwide demand for it is presumed to be over 10,000 tons before 1995.

For industrial production of α -APM, for example, the following methods are known. Precisely, they are (1) a method of obtaining α -APM in which an N-substituted aspartic acid anhydride and a phenylalanine methyl ester are bonded to each other in an organic solvent and the substituent is removed from the product by an ordinary method (U.S. Patent 3,786,039); (2) a method of obtaining α -APM in which α -L-aspartyl-L-phenylalanine is methyl-esterified in a mixed solvent comprising water, methanol and hydrochloric acid to obtain α -APM hydrochloride, and this is neutralized to obtain α -APM (Japanese Patent Application Laid-Open No. 53-82752); and (3) a method of obtaining α -APM in which an N-substituted aspartic acid and a phenylalanine methyl ester are condensed in the presence of an enzyme and thereafter the substituent is removed from the product (Japanese Patent Application Laid-Open No. 55-135595).

In the above-mentioned chemical synthetic method (1), a β -isomer ( β -L-aspartyl-L-phenylalanine methyl ester) is produced essentially as a side product. As a means of selectively removing impurities including the said β -isomer, there is known (4) a purification method in which α -APM containing impurities is brought into contact with a hydrohalogenic acid and then subjected to solid-liquid separation so as to isolate α -APM as its hydrohalide salt.

Where industrial scale production of α -APM is taken into consideration so as to meet the trend of the demands of the current time, chemical methods are the major methods from the viewpoint of the manufacture costs. In this case, esterification is often effected via its hydrochloride as in the above-mentioned method (2), or after the formation of α-APM, it is often formed into its hydrohalide salt such as the hydrochloride for purifying it as in method (4). For obtaining α -APM from its hydrohalide salt such as its hydrochloride by an ordinary method, a hydrohalide salt of α -APM is dissolved or suspended in an aqueous medium and the resulting solution or suspension is neutralized by adding an aqueous solution of a base such as sodium hydroxide, sodium hydrogen carbonate or ammonia.

However, the present inventors found that when a large amount of a liquid in a liter scale or more must be dealt with especially in the industrial scale neutralization of such an acid addition salt of α -APM, the conventional neutralization method has serious problems such as those mentioned below.

Precisely, where a base is continuously dropwise added to an aqueous solution of an acid addition salt of α -APM to the isoelectric point of α -APM, rapid precipitation of α -APM will occur during the course of the addition so that stirring of the system will become impossible. In the worst case, it has been found that the stirrer stops. If the liquid amount is at most 100 ml or so in a laboratory scale experiment, the precipitated solid phase may easily be broken with a tool such as a spatula by an experimentalist whereby the fluid condition may well be recovered. However, where a large amount of a liquid in a liter scale or more, for example, in a so-called bench plant or the like, or where the neutralization is carried out in a large-scaled pilot plant or commercial plant, such a means cannot be used for solving the problem.

As a countermeasure to the problem in the industrial scale neutralization, addition of a large amount of water may be considered so as to carry out the neutralization under diluted concentration condition. However, this causes an extreme lowering of the capacity efficiency of the device used and also causes extreme lowering of the yield of the product. Therefore, the means could not be said to be good. On the other hand, extremely slow addition of an aqueous base solution over an extremely long period of time will be effective for ensuring the fluidity of the liquid, which, however, is extremely ineffective from the viewpoint of the producibility. In addition, still another method of intermittently discontinuing the dropwise addition of the neutralizing agent to the reaction system of having a pH range at which precipitation of α -APM starts or a pH value of being 2.5 or so to thereby ripen the precipitated crystals may be employed (Japanese Patent Application Laid-Open No. 63-145298). However, the method also has the serious drawback that the pH range suitable for the ripening will greatly fluctuate if the initial concentration of α -APM (or its acid addition salt such as its hydrochloride) is strictly controlled to be a certain value. It is always the case that the α-APM content in the separated wet crystals (acid addition salt) fluctuates every time, depending upon the delicate condition in crystallizing the acid addition salt of α -APM in the pre-step. In such a case, it is difficult to keep the initial concentration of α -APM (or its acid addition salt such as its hydrochloride) constant in a disssolution system to be operated under control of the liquid amount which is often employed in industrial production. In order to evade the problem, a complicated concentration control is necessary to effect batchwise analysis every time to supply crystals or water, when needed; or an expert monitor who is skilled in the art must be exclusively engaged to the system so as to be able to batchwise determine the suitable ripening pH value every time.

In addition, even though the problems of the operation could be evaded by any of the above-mentioned systems, the α -APM crystals to be obtained still have an extremely poor solid-liquid separatability so that the crystals cause a noticeable increase of the necessary equipment and energy in the filtration and drying step as the post-treatment step.

The present inventors made earnest and repeated investigations for the purpose of overcoming the above-mentioned problems in the neutralization step of neutralizing an acid addition salt of α -APM and, as a result, have obtained the following findings.

Specifically, in dissolving an acid addition salt of α -APM in an aqueous medium, the α -APM concentration in the solution at the end of the neutralization of the same is defined to fall within a particular range and thereafter the solution is heated up to an elevated temperature and subjected to neutralization to the isoelectric point of α -APM with stirring, then the resulting neutralized liquid is cooled to cause the precipitation of crystals of α -APM. In such a way, they have found that the above-mentioned problems of the operation and filtration can be overcome without the necessity of any skill in carrying out the process.

Cooling of the neutralized liquid may subsequently be effected under the condition of a forced flow, for example, with stirring to obtain a crystal suspension with fluidity, which is generally called a slurry. Alternatively, cooling may also be effected rapidly with, with extremely slow or without stirring to obtain all or a part of the liquid as a non-fluid sherbet-like pseudo solid phase. It has been found that the latter is more effective from the viewpoint of improving the operatability of the process and the solid-liquid separatability of the crystals formed.

For improving the filterability of the crystals formed, the present inventors have found that the main reason for worsening the filterability is the crystallization inhibiting effect of the co-existing impurities such as salts formed by neutralization. Therefore, before initiating cooling, α -APM having a relatively high purity is added to the neutralized liquid so as to reduce the relative concentration of the impurities, or de-salting by recrystallization or electric dialysis or with a reversed osmotic membrane may additionally be effected prior to cooling, whereby noticeable improvement of the filterability of the crystals formed has been attained. In addition, it has also been found that the powdery properties of the dried final product may be improved noticeably.

The present inventors have applied such findings to an industrial process of producing α -APM and have solved all the above-mentioned problems of the prior art. As a result, they have achieved drastic rationalization of the process of producing α -APM, including reduction of the necessary equipment and energy and reduction of the number of the workers necessary for carrying out the process. Thus, they have hereby completed the present invention.
The present invention relates to a method for preparing α-L-aspartyl-L-phenylalanine methyl ester by neutralizing in an aqueous medium an acid addition salt of α-L-aspartyl-L-phenylalanine methyl ester with a base to the isoelectric point, characterized in that
(a) the concentration of α-L-aspartyl-L-phenylalanine methyl ester is controlled to fall within the range of from 3 to 10 weight-% at the time of completion of the neutralization of the ester,
(b) the neutralization is effected with stirring and i) while maintaining an elevated temperature of the liquid within the range of 50 to 80 °C during the neutralization, or ii) raising the temperature to a value within the range of 50 to 80 °C after the neutralization,
(c) the neutralized liquid resulting from step (b) i) or ii) is subsequently cooled so that crystals of α-L-aspartyl-L-phenylalanine methyl ester are precipitated out.

Acid addition salts of α -APM usable in the present invention may be mineral acid salts such as hydrochloride, hydrobromide, sulfate and phosphate of it. Especially, the hydrochloride is often used.

In the present invention, an acid addition salt of α -APM is neutralized in the form of an aqueous solution or suspension. As the solvent, suitable is water or a mixed solvent comprising a water-miscible organic solvent and water. Since the solubility of α -APM (acid addition salt) temporarily increases with neutralization, all the acid addition salt crystals need not to be dissolved at the time of the start of neutralization.

As a base as a neutralizing agent, usable are alkali hydroxides such as sodium hydroxide, alkali carbonates or bicarbonates such as sodium carbonate, ammonia and other organic amines. From the viewpoint of prevention of decomposition of α -APM during neutralization as well as of the cost and the easy operatability, sodium carbonate or ammonia is frequently used in the form of its aqueous solution. The amount of the base to be used is such as being necessary for making the solution of the acid addition salt of α -APM to reach the isoelectric point of α -APM.

In neutralization, close attention must be paid to the determination of the operating temperature and the α -APM concentration also in consideration of the increment of the water content to be caused by addition of a neutralizing agent. That is to say, if the concentration is too low, the yield in the crystallization will be low. After investigation, it has been found that when the initial concentration is 3 % or less as α -APM in crystallization , then the filterability of the crystals formed rapidly worsens. Therefore, the α -APM concentration at the time of the start of crystallization must be 3 % or more and the operating temperature must be 50 ° C or higher at which almost no precipitation of crystals is admitted from a solution of this concentration. On the contrary, if the concentration is too high, excess crystals will precipitate in neutralization. Although precipitation of a small amount of crystals at the end of the neutralization would not noticeably interfere with the advantage of the present invention, it is preferred that no crystals precipitate at the end of the neutralization or at least at the start of the crystallization for the purpose of most effectively obtaining the best effect of the present invention. Therefore, where the α -APM concentration is set high at the start of crystallization, the liquid temperature must thereby be higher in accordance with the high α -APM concentration. However, α -APM has a stability problem when it is dissolved in an aqueous medium. That is, an aqueous α -APM solution will form α -L-aspartyl-L-phenylalanine (hereinafter referred to as " α -AP") or a diketopiperazine compound (hereinafter referred to as "DKP") by hydrolysis or an intramolecular cyclization reaction under high temperature conditions. Such decomposition reaction lowers the yield and the quality of the product and is therefore unfavorable . From the viewpoint of inhibiting the decomposition, the upper limit of the temperature is desirably defined to be 80 ° C and that of the α -APM concentration to be 10 % or less which is the solubility at this temperature. However, in the low pH range where a large amount of free acid is present during the course of neutralization, α -AP is often formed by hydrolysis under high temperature conditions. In this case, therefore, it is desired that the liquid temperature is 40 ° C or lower until the pH value is slightly above the isoelectric point of the acid addition salt of α -APM (for instance, in the case of hydrochloride, pH of 2.5 or so). Since the solubility in the vicinity of the isoelectric point of the acid addition salt of α -APM is noticeably high (for instance, in the case of α -APM hydrochloride, it has a solubility of 10 % or more at a temperature of 30 ° C), there is no danger of precipitation of crystals at this stage. Afterwards, neutralization may be effected continuously up to the isoelectric point of α -APM during or after elevation of the temperature. Where an aqueous sodium carbonate solution or the like is used as a neutralizing agent, it is preferred to keep the neutralized liquid in high temperature conditions within the range of not remarkably promoting decomposition of α -APM after neutralization, since such a high temperature condition may promote release of carbon dioxide gas from the liquid and may make the operation of the crystallization step easier.

In the next crystallization step, the same apparatus as that used in the neutralization step may be used. However, from the viewpoint of the materials of constituting the apparatus, employment of another apparatus made of different lower quality materials would be preferred for saving the plant costs. For instance, stirring tank type, draft tube baffle type or crystal-Oslo type crystallizers as well as modified ones thereof which are widely and popularly used as an industrial crystallizer, can be used. Such crystallizers may be driven under a forced flow condition, in accordance with the intrinsic operation of them, whereby a fluid crystal floating suspension of a so-called slurry can be obtained by cooling. As a matter of course,this case is superior to the other case where crystals are precipitated out directly by neutralization in point of the easy operation of the process and the good filterability of the crystals formed. In order to further improve the process, cooling is desirably effected without stirring or with intermittent stirring or under a weak forced flow whereby the whole or a part of the neutralized liquid is obtained as a sherbet-like pseudo solid phase. In this case, however, the time necessary for the cooling would be longer than that for the case of effecting a sufficient forced flow and, in addition, the inside of the crystallizer would have a temperature distribution. Therefore, from the viewpoint of an inhibition of decomposition of α -APM and of a promotion of the producibility of it, attention should be paid so that the maximum distance between the substance to be cooled and the cooling surface in the crystallizer is not too large. Therefore, in this case, the above-mentioned conventional industrial crystallizers could be used under the driving condition with no stirring or with weak stirring or intermittent stirring, but more preferably, a crystallizing apparatus as planned in such a way that a sufficient large heat transfer surface is ensured to the liquid amount and that the maximum distance between the substance to be cooled and the cooling surface is 500mm or less is used. After formation of the pseudo solid phase, cooling may be continued as it is and/or the pseudo solid phase formed is broken under the condition of a forced flow and, if desired, cooling is further effected, whereupon the residual α -APM as remained in the mother solution may be recovered.

In accordance with the above-mentioned method, almost all the problems on operation of the neutralization step for neutralizing an acid addition salt of α -APM and the crystallization step for forming crystals of α -APM could be solved. In addition, the solid-liquid separatability of the finally obtained α -APM crystals as well as the powdery properties of the dried product of them may noticeably be improved further by selectively removing the impurities as having been in the pre-step before crystallization. Impurities which seriously interfere with the growth of crystals of α -APM, are the salts formed by neutralization. Various salts are formed by neutralization, depending upon the kinds of the acids and bases used. For instance, where hydrochloric acid is used as an acid and sodium carbonate is used as a base, sodium chloride is formed; and where hydrobromic acid is used as an acid and ammonia is used as a base, ammonium bromide is formed. Any of such salts has almost the same severe effect of interfering with the crystallization of α -APM. For instance, only when about 1 % of such a salt is in the liquid to be crystallized, it will have an influence on the growth of crystals of α -APM in crystallization.

For selectively removing such salts and other impurities, for example, recrystallization is effective. Precisely, the crystals to be obtained by the first crystallization to be effected directly after neutralization are again dissolved in a hot aqueous medium and the resulting solution is cooled to precipitate crystals. In both, the first crystallization and the second crystallization, any of the above-mentioned crystallization systems may be employed. Preferably, however, the method of forming a sherbet-like pseudo solid phase is employed for at least the second crystallization.

It is also effective to subject the neutralized liquid to Ruth's reverse osmotic membrane treatment or electric dialysis treatment for desalting it, since the crystallization may be finished by one operation. In the case, desalting of the neutralized liquid and concentration of it may be carried out simultaneously, and the final α -APM concentration may be well within the range of from 3 to 10 %. As the case may be, the concentration may be lower than this range at the end of the neutralization. Desalting is especially preferably conducted in such a way that at least 50 % or more of salts existing at the start of the operation may be removed, in view of improvement of the solid-liquid separatability of the crystals formed.

Improvement of the crystal property of α -APM may also be effected without removing salts from the system. Salts express their function as a relative ratio to the α -APM concentration in the system. Therefore, a solution of α -APM having a relatively high purity and/or crystals thereof are added to the neutralized liquid (or solution of α -APM acid addition salt) before, during or after neutralization, and if desired, water is added thereto, then blended and dissolved to form a solution having an α -APM concentration of from 3 to 10 %. The resulting solution is then cooled for crystallization. α -APM to be added may derive from any origins. More efficiently, a part of the α-APM crystals as isolated by the method of the present invention may be used as the additional α -APM component. While they are still in a wet condition, they may be used for neutralization of an acid addition salt of α -APM in the next batch. The amount of such α -APM crystals may be larger to obtain a higher effect. From the viewpoint of the producibility, however, it is suitably not larger than the molar amount of the acid addition salt of α -APM to be neutralized.

In accordance with the method of the present invention of preparing α -APM by neutralizing an acid addition salt of α -APM with a base in an industrial scale, especially in a liter scale or larger, the operatability of the liquid such as the fluidity of the liquid during neutralization is extremely improved and the solid-liquid separatability of the α -APM crystals to be produced as well as the powdery characteristics of the dried product from them is noticeably improved. Additionally, the steps of constituting the method are simplified. Therefore, the method of the present invention is extremely valuable for industrial use.

### Examples:

Next, the present invention will be explained in more detail by way of the following examples.

The test of evaluating the filterability of α -APM crystals as obtained in the examples was effected by the method mentioned below.

### Method of Measuring Filtration Specific Cake Resistance

One liter of a sample to be tested is sampled and is filtered through a top-feed system suction filter (leaf tester). The pressure difference in filtration is 70 mmHg, which is kept constant throughout the term of filtration. From the start of filtration, the amount of the filtrate V[cm³] is measured at regular intervals and plotted on a graph having the amount of the filtrate as the horizontal axis and the value ( θ /V) as obtained by dividing time of filtration θ [s] by the amount of the filtrate as the vertical axis. The inclination of the line K[s/ml²] is obtained by the minimum square method. The value C' as obtained by dividing the total amount [g] of the crystals in the slurry by the total amount [cm³] of the liquid in the slurry is introduced into the following equation. The filtration area A is 93 [cm²]; and the viscosity µ of the filtrate is 0.0135 [g/cm.s]. The specific cake resistance α thus calculated out is a criterion for the filterability of the sample. The sample having a smaller value α is more easily filterable.

### Equation of Specific Cake Resistance :

${\text{α = 20.K.A}}^{\text{2}} \text{.PT/ µ.C' [m/kg]}$ where α is a specific cake resistance [m/kg] of the filtered cake;
µ is a viscosity of the filtrate [g/cm.s ;
PT is a pressure difference [dyne/cm²] by the filtered cake and the filtration device = A P[mmHg] × 1333.22;
A is filtration area [cm²]; and
C' is a weight of the crystals per the unit volume of the liquid component in the slurry [g/cm³] = dry cake weight [g]/(wet cake weight [g] - dry cake weight [g] + amount of final filtrate [cm³]).

### Example 1:

1900 ml of ion-exchanged water were put in a 2-liter jacket-combined separable flask having an inner diameter of 125 mm, this was stirred at 250 rpm with an anchor-type stirrer having a blade diameter of 10 cm as set to the flask. The inside temperature of the flask was kept to be 27 ° C by circulating warm water into the jacket, and 150 g of wet crystals of APM hydrochloride were dissolved. To this was added a 150 mM Na₂CO₃ aqueous solution so that the liquid blend was adjusted to have a pH of 2.5 and then heated up to 55 ° C. Further, it was adjusted to have a pH of 4.9 with Na₂CO₃ aqueous solution and was heated up to 65 ° C. The α -APM concentration was 4.2 g/dl. With continuously stirring at 250 rpm, stirring crystallization was effected while a cooling medium of 5°C was introduced into the jacket. Cooling was continued until the temperature of the system became 9 ° C. During the course of cooling, no worsening of the stirring conditions due to the deposition of crystals was admitted and a good operation was ensured throughout the cooling procedure. Thus, a slurry with good solid-liquid separatability was obtained, having a filtration specific cake resistance of 4.6 × 10¹⁰ m/kg.

### Comparative Example 1-1:

The same apparatus as that used in Example 1 was used and stirring was effected at 250 rpm. The inside temperature was kept to be 27 ° C with introducing warm water into the jacket, and 240 g of wet crystals of APM hydrochloride were dissolved in 1900 ml of water. The α -APM concentration was 7.3 g/dl. While the solution was kept at a temperature of 27 ° C, 16 % sodium carbonate was dropwise added thereto at a constant rate (7 ml/min). The scheduled time before the end of the neutralization (pH 4.9) was 30 minutes, but the liquid lost the fluidity due to the deposition of crystals (pH 3.0, after 16 minutes), and the stirring stopped 21 minutes after the start of the experiment. The content in the flask was solidified.

### Comparative Example 1-2:

Using the same apparatus as that used in Example 1, 150 g of crystals of APM hydrochloride were dissolved in 1900 ml of water and heated up to 28 ° C. The solution contained 4.6 g/dl of α -APM. With stirring at 400 rpm, 130 ml of a 150 mM Na₂CO₃ aqueous solution were dropwise added to the solution with a micro-pump over a period of 6 hours so that the solution was neutralized to have a final pH of 4.9. Rapid deposition of crystals occurred when the pH of the solution became 3.1 or so and stirring of the liquid became difficult but could still hardly be effected. After cooled to 9 ° C, the obtained slurry was dewatered in a centrifugal separator having a diameter of about 12 cm at 3800 rpm for 5 minutes. Handling of the slurry was extremely difficult, as shown by a filtration specific cake resistance of 1.1 × 10¹¹ m/kg. The moisture content in the crystals was 65 %.

### Example 2:

1900 ml of ion-exchanged water were put in a 2-liter jacket-combined separable flask having an inner diameter of 125 mm, this was stirred at 250 rpm with an anchor-type stirrer as set to the flask. The inside temperature of the flask was kept to be 28 ° C by circulating warm water into the jacket, and 150 g of wet crystals of APM hydrochloride were dissolved.

To this was added a 150 mM Na₂CO₃ aqueous solution so that the liquid blend was adjusted to have pH of 2.5 and then heated up to 55 ° C. Further, it was adjusted to have a pH of 4.9 with the Na₂CO₃ aqueous solution and was heated up to 65 ° C. The α -APM concentration was 4.6 g/dl . After the stirring was stopped, a cooling medium of 5°C was introduced into the jacket. Cooling was continued without stirring, and the whole of the neutralized liquid became a sherbet-like phase. Next, this was stirred at 200 rpm and cooling was continued further until the inner temperature of the system became 7 ° C. The slurry thus obtained was dewatered in a centrifugal separator having a diameter of 4.7 inches at 3800 rpm for 5 minutes. The slurry was extremely good, having a specific cake resistance of 2.8 x 10⁹m/kg.The separatability of the crystals formed was also good, having a moisture content therein of 47 %.

### Example 3:

1900 ml of an aqueous solution of APM hydrochloride were put in the same apparatus as that used in Example 2, and 28 % NH₄OH was added thereto with stirring at 200 rpm at 27 ° C so that the solution was adjusted to have a pH of 3.0. The solution was heated up to 55 ° C and 28 % NH₄OH was added thereto to yield a pH of 4.9. The decolored liquid contained 4.2 g/dl of α -APM. After the stirring was stopped. A cooling medium of 5°C was introduced into the jacket so as to cool the system. Thus, a sherbet-like phase was formed wholly in the inside of the flask. After 50 minutes, stirring was again started at 200 rpm and cooling was continued until the inner temperature became 7.5 ° C. The slurry thus obtained was dewatered in a centrifugal separator having a diameter of 4.7 inches at 3800 rpm for 5 minutes.

The slurry had a good solid-liquid separatability, having a filtration specific resistivity of 3.1 x 10⁹ m/kg and the moisture content of the obtained crystals was 46 %.

### Comparative Example 2:

Using the same apparatus as that used in Example 2, 65 g of crystals of APM hydrochloride were dissolved in 1760 ml of water. The solution was neutralized in the same manner as in Example 2 with a 150mM Na₂CO₃ aqueous solution and then decolored with active charcoal. The solution was analyzed to contain α -APM of 2.4 g/dl. The aqueous solution was heated up to 68 ° C and left as it was for one hour. Then, the stirring was stopped, and a cooling medium of 5°C was introduced into the outer jacket for conducting static crystallization. Cooling was continued for 3 hours without stirring, then stirring was effected at 200 rpm and cooling was continued further until the inner temperature became 7 ° C. The slurry thus obtained was dewatered in a centrifugal separator having a diameter of 4.7 inches at 3800 rpm for 5 minutes. The slurry was much worse than that obtained in Example 2, by exhibiting a filtration specific resistivity of 1.3 × 10¹⁰ m/kg and the moisture content of the filtered crystals was 72 %.

### Example 4:

This demonstrates a 400-liter scale experiment. Precisely, 24 kg of crystals of APM hydrochloride were dissolved in 320 liters of water and heated up to 36 ° C. With stirring, 1.3 liters of a 28 % NH40H aqueous solution were added thereto. whereby the resulting solution was adjusted to a pH of 2.5. This was further heated up to 65.5 ° C, and 3.0 liters of a 28 % NH₄0H aqueous solution were added thereto to yield a pH of 4.9. The aqueous solution contained 4.9 g/dl of α -APM and this was transferred into a jacket-combined cylindrical crystallizer having an inner diameter of 400 ml and a total length of 3000 mm. The crystallizer had no stirring equipment. To the jacket, a cooling medium of -5°C was introduced for 3.5 hours. The bottom of the crystallizer was opened and the liquid content was transferred into another crystallizer equipped with a stirring equipment. This was continuously cooled overnight with stirring until the temperature thereof became 5 ° C. The bottom of the crystallizer was opened and the crystals were taken out therefrom, whereupon no crystal scaling on the inner wall of the crystallizer was observed and the crystal slurry was taken out very well. 350 liters of the slurry thus obtained was subjected to solid-liquid separation in a centrifugal separator having a diameter of about 91 cm and a volume of 92 liters, whereupon only 3 minutes were needed for charging the slurry into the separator. The slurry was then dewatered therein at 1100 rpm and 600 G for 30 minutes. The slurry was extremely good, having a filtration specific cake resistance of 1.7 x 10⁹ m/kg and the moisture content of the filtered crystals was 38 %.

### Example 5:

Using a 700-liter crystallizer equipped with a stirring equipment and a jacket, 25 kg of wet crystals of APM hydrochloride were dissolved in 300 liters of water as previously warmed up to 35 ° C. With stirring, 5 liters of a 15 % Na₂CO₃ aqueous solution were added thereto yield a pH of 2.5. After this was heated up to 60 ° C, 29 liters of a 15 % Na₂CO₃ aqueous solution were further added thereto to make pH of 4.9. This was heated up to 65.5 ° C and, with stirring, a cooling medium of -5°C was introduced into the jacket so that stirring crystallization was continued overnight under the condition. The slurry thus obtained had a filtration specific cake resistance of 4.0 x 10¹⁰ m/kg. This was dewatered in a centrifugal separator having a diameter of about 12 cm at 3800 rpm for 5 minutes, and the crystal moisture content thereof was 50 %.
35 kg of the thus obtained crude wet crystals were again dissolved in 300 liters of water previously heated up to 65 ° C. The resulting solution contained 5.0 g/dl of α -APM. This was transferred into the same cylindrical jacket-combined crystallizer having an inner diameter of 400 mm and a total length of 3000 mm as that used in Example 4, and a cooling medium of -5°C was introduced into the jacket for 3.5 hours. The bottom of the crystallizer was opened and the liquid content was transferred into another crystallizer equipped with a stirring equipment. This was continuously cooled overnight with stirring until the temperature thereof became 3 ° C. The bottom of the crystallizer was opened and the crystals were taken out therefrom, whereupon no scaling on the inner wall of the crystallizer was observed and the crystal slurry was taken out very well 330 liters of the slurry thus obtained was subjected to solid-liquid separation in a centrifugal separator having a diameter of about 91 cm and a volume of 92 liters, whereupon only 3 minutes were needed for charging the slurry into the separator. The slurry was then dewatered therein at 1100 rpm and 600 G for 30 minutes.

The slurry was extremely good, having a filtration specific cake resistance of 2.7 × 10⁸ m/kg and the moisture content of the obtained crystals was 28 %.

### Example 6:

1050 ml of an aqueous solution of APM hydrochloride were put in the same apparatus as that used in Example 2, and 28 % NH₄0H was added thereto with stirring at 200 rpm at 27 ° C to yield a pH of 2.5. The solution was heated up to 55 ° C and 28 % NH₄0H was added thereto to yield a pH of 4.9. The crystals formed were taken out in the same manner as in Example 1.

A part (28 g) of the α -APM crystals formed was dissolved in 365 ml of deionized water and the resulting solution was added to the neutralized liquid to obtain an aqueous solution containing 5.2 g/dl of α -APM. The aqueous solution was heated up to 68 ° C and the stirring was stopped. Then, a cooling medium of 5 ° C was introduced into the jacket. Cooling was continued for one hour without stirring, then stirring was effected at 200 rpm, and cooling was thus continued under the stirring condition until the inner temperature became 7 ° C. The slurry thus obtained was dewatered in a centrifugal separator having a diameter of 4.7 inches at 3800 rpm for 5 minutes.

The slurry was extremely good, having a specific cake resistance of 2.4 x 10⁹ m/kg and the moisture content of the obtained crystals was 35 %.

### Example 7:

700 ml of an aqueous solution of APM hydrochloride were put in the same apparatus as that used in Example 2, and 28 % NH₄OH was added thereto with stirring at 200 rpm at 27 ° C to yield a pH of 2.5. The solution was heated up to 55 ° C and 28 % NH₄0H was added thereto to yield a pH of 4.9.
56 g of the α-APM crystals obtained in the same manner as in Example 1 were dissolved in 730 ml of deionized water and the resulting solution was added to the neutralized liquid to obtain an aqueous solution containing 4.9 g/dl of α -APM. The aqueous solution was heated up to 68 ° C and the stirring was stopped. Then, a cooling medium of 5°C was introduced into the jacket for conducting static crystallization. Precisely, cooling was continued for one hour without stirring, then stirring was effected at 200 rpm, and cooling was thus continued under the stirring condition until the inner temperature became 7 ° C. The slurry thus obtained was easily separated by solid-liquid separation and was extremely good, having a specific cake resistance of 1.1 x 10⁹ m/kg and a moisture content of the obtained crystal of 34 %.

### Example 8:

This demonstrates a 30-liter scale experiment.

Precisely, 1200 g of α -APM hydrochloride was put in 30 liter of water and dissolved with stirring at 30 ° C. With still stirring, 155 ml of a 24 wt.% Na₂CO₃ aqueous solution were added to the solution to yield a pH of 2.5.Then, the solution was heated up to 50 ° C. 595 ml of a 24 wt.% Na₂CO₃ aqueous solution were again added thereto to yield a final pH of the solution of 4.9.
The α -APM concentration in the neutralized liquid was 2.2 %, and the NaCl concentration therein was 0.76 %.

With maintaining the temperature at 50 ° C, the neutralized liquid was subjected to Ruth's reverse osmotic filtration. As the osmotic membrane for the filtration used was a spiral model Ruth's reverse osmotic membrane module manufactured by Teijin Engineering Co. (membrane area 2.4 m²). The neutralized liquid as stocked in a stock tank was fed to the membrane module at an operating pressure of 14 kgf.cm⁻² and was subjected to batch-wise desalting in such a way that the liquid as passed through the membrane was taken out of the system while the liquid not passed therethrough was returned back to the stock tank. At the point when the liquid amount in the stock tank became 1/1.5 of the initial amount, 10 liters of water were added thereto so that the whole amount was again made to be 30 liters. Thereafter the dialysis filtration was further effected in the same manner until the liquid amount therein became 1/1.5 of the recovered amount. Finally, 55 % of NaCl as initially existing in the neutralized liquid was removed. The recovery of α -APM was 96 %. The volume of the liquid after the Ruth's reverse osmotic filtration treatment was 20 liters, which had an α -APM concentration of 3.2 % and an NaCl concentration of 0.51 %.

The desalted liquid thus obtained was put in a container having a diameter of 350 mm and was heated up to 65°C. Using a cooling medium of 5°C, this was subjected to static crystallization without stirring for 2 hours. The content in the container became almost wholly a sherbet-like phase. This was stirred with a stirrer for one hour and cooled to a final temperature of 5 ° C. The crystal slurry thus obtained had a specific cake resistance of 1.5 x 10⁹ m/kg. This was dewatered in a centrifugal separator having a diameter of about 12 cm at 3800 rpm for 5 minutes to obtain wet crystals. They had a moisture content of 53 %. The solid-liquid separatability of the slurry was extremely good.

## Claims

1. A method for preparing α-L-aspartyl-L-phenylalanine methyl ester by neutralizing in an aqueous medium an acid addition salt of α-L-aspartyl-L-phenylalanine methyl ester with a base to the isoelectric point, characterized in that
(a) the concentration of α-L-aspartyl-L-phenylalanine methyl ester is controlled to fall within the range of from 3 to 10 weight % at the time of completion of the neutralization of the ester,
(b) the neutralization is effected with stirring and
i) while maintaining an elevated temperature of the liquid within the range of 50 to 80°C during the neutralization, or
ii) raising the temperature to a value within the range of 50 to 80°C after the neutralization,
(c) the neutralized liquid resulting from step (b) i) or ii) is subsequently cooled so that crystals of α-L-aspartyl-L-phenylalanine methyl ester are precipitated out.

2. The method as claimed in claim 1, in which the neutralization is effected at a pH of 2.5 and a liquid temperature of 40°C or lower and thereafter the neutralization is continued during or after elevation of the liquid temperature to a maximum of 80°C.

3. The method as claimed in claim 1 or 2, in which cooling of the neutralized liquid is effected under the condition of a forced flow to obtain a flowing crystal suspension with fluidity.

4. The method as claimed in any of the claims 1 or 2, in which cooling of the neutralized liquid is effected without forced flow or under a weak forced flow so that all or a part of the liquid is obtained as a sherbet-like pseudo solid phase.

5. The method as claimed in any of the claims 1 to 4, in which a solution and/or crystals of α-L-aspartyl-L-phenylalanine methyl ester are added before, during or after neutralization and, if desired, water is further added, blended and dissolved, then after the concentration of the resulting solution is adjusted to fall within a range of from 3 to 10 %, cooling of the solution is effected.

6. The method as claimed in any of the claims 1 to 5, in which the neutralization is followed by de-salting by means of dialysis.

7. The method as claimed in claim 6, in which 50% or more of the salt existing at the end of the neutralization step is removed.

8. A method as claimed in any of the claims 1 to 7, characterized in that
the α-L-aspartyl-L-phenylalanine methyl ester obtained after the crystallisation step (c) is again dissolved, and the resulting solution is cooled with or without agitation.

9. The method as claimed in claim 8, wherein the cooling is carried out without agitation or under a weak forced flow so that all or a part of the liquid is obtained as a sherbet-like pseudo solid phase.

10. The method as claimed in any of the claims 1 to 8, in which the acid addition salt of α-L-aspartyl-L-phenylalanine methyl ester is the hydrochloride of the same.

## Patentansprüche

1. Verfahren zur Herstellung von α-L-Aspartyl-L-phenylalanin-methylester durch Neutralisation eines Säureadditionssalzes von α-L-Aspartyl-L-phenylalanin-methylester in einem wäßrigen Medium mit einer Base bis zum isoelektrischen Punkt, dadurch gekennzeichnet, daß
(a) die Konzentration von α-L-Aspartyl-L-phenylalanin-methylester so eingestellt wird, daß sie zum Zeitpunkt der Beendigung der Neutralisation des Esters im Bereich von 3 bis 10 Gew.-% liegt,
(b) die Neutralisation unter Rühren durchgeführt wird, wobei
i) während der Neutralisation eine erhöhte Temperatur der Flüssigkeit im Bereich von 50 bis 80°C aufrecht gehalten wird oder
ii) die Temperatur nach der Neutralisation auf einen Wert im Bereich von 50 bis 80°C erhöht wird,
(c) die aus Stufe (b) i) oder ii) resultierende neutralisierte Flüssigkeit anschließend so abgekühlt wird, daß Kristalle von α-L-Aspartyl-L-phenylalanin-methylester abgeschieden werden.

2. Verfahren nach Anspruch 1, wobei die Neutralisation bei einem pH von 2,5 und einer Flüssigkeitstemperatur von 40°C oder weniger durchgeführt wird und danach die Neutralisation während oder nach der Erhöhung der Flüssigkeitstemperatur bis zu einem Maximalwert von 80°C fortgesetzt wird.

3. Verfahren nach Anspruch 1 oder 2, bei dem das Abkühlen der neutralisierten Flüssigkeit unter der Bedingung einer Zwangsströmung durchgeführt wird, so daß eine fließende Kristallsuspension mit Fluidität erhalten wird.

4. Verfahren nach einem der Ansprüche 1 oder 2, bei dem das Abkühlen der neutralisierten Flüssigkeit ohne Zwangsströmung oder unter einer schwachen Zwangsströmung durchgeführt wird, so daß die gesamte Flüssigkeit oder ein Teil dieser in Form einer sorbetartigen Pseudofestphase erhalten wird.

5. Verfahren nach einem der Ansprüche 1 bis 4, bei dem eine Lösung und/oder Kristalle von α-L-Aspartyl-L-phenylalanin-methylester vor, während oder nach der Neutralisation zugesetzt wird/werden und gewünschtenfalls außerdem Wasser zugefügt wird, gemischt und gelöst wird, wobei danach, nachdem die Konzentration der resultierenden Lösung so eingestellt wurde, daß sie im Bereich von 3 bis 10 % liegt, das Abkühlen der Lösung durchgeführt wird.

6. Verfahren nach einem der Ansprüche 1 bis 5, bei dem auf die Neutralisation ein Entsalzen mit Hilfe von Dialyse folgt.

7. Verfahren nach Anspruch 6, bei dem 50 % oder mehr des am Ende der Neutralisationsstufe vorliegenden Salzes entfernt werden.

8. Verfahren nach einem der Ansprüche 1 bis 7, dadurch gekennzeichnet, daß der nach der Kristallisationsstufe (c) erhaltene α-L-Aspartyl-L-phenylalanin-methylester erneut gelöst wird und die resultierende Lösung mit oder ohne Rühren gekühlt wird.

9. Verfahren nach Anspruch 8, wobei das Kühlen ohne Rühren oder unter einer schwachen Zwangsströmung durchgeführt wird, so daß die Gesamtmenge oder ein Teil der Flüssigkeit in Form einer sorbetartigen Pseudofestphase erhalten wird.

10. Verfahren nach einem der Ansprüche 1 bis 8, bei dem das Säureadditionssalz des α-L-Aspartyl-L-phenylalanin-methyl-esters dessen Hydrochlorid ist.

## Revendications

1. Procédé de préparation de l'ester méthylique d'α-L-aspartyl-L-phénylalanine par neutralisation dans un milieu aqueux d'un sel d'addition acide de l'ester méthylique d'α-L-aspartyl-L-phénylalanine avec l'aide d'une base jusqu'au point isoélectrique, caractérisé en ce que
(a) la concentration d'ester méthylique d'α-L-aspartyl-L-phénylalanine est contrôlée de manière à se trouver dans l'intervalle de 3 % jusqu'à 10 % en poids à la fin de la neutralisation de l'ester,
(b) la neutralisation est réalisée sous agitation et i) tout en maintenant une température élevée du liquide dans l'intervalle de 50 °C jusqu'à 80 °C au cours de la neutralisation, ou ii) en élevant la température jusqu'à une valeur dans l'intervalle de 50 °C jusqu'à 80 °C dans des conditions d'écoulement forcé après la neutralisation,
(c) le liquide neutralisé résultant de l'étape (b) i) ou ii) est ensuite refroidi de manière à ce que les cristaux d'ester méthylique d'α-L-aspartyl-L-phénylalanine soient éliminés par précipitation.

2. Procédé selon la revendication 1, dans lequel la neutralisation est réalisée à un pH de 2,5 et à une température du liquide de 40 °C ou inférieure, la neutralisation étant ensuite poursuivie au cours ou après élévation de la température du liquide jusqu'à un maximum de 80 °C.

3. Procédé selon la revendication 1 ou 2, dans lequel le refroidissement du liquide neutralisé est réalisé dans des conditions d'écoulement forcé afin d'obtenir une suspension cristalline s'écoulant avec fluidité.

4. Procédé selon l'une quelconque des revendications 1 ou 2, dans lequel le refroidissement du liquide neutralisé est réalisé en l'absence d'écoulement forcé ou sous un écoulement forcé faible de manière à obtenir que tout ou partie du liquide soit sous forme de pseudo-phase solide de type sherbet.

5. Procédé selon l'une quelconque des revendications 1 à 4, dans lequel on ajoute une solution et / ou des cristaux d'ester méthylique d'α-L-aspartyl-L-phénylalanine avant, pendant ou après, la neutralisation avec addition éventuelle d'eau, qu'on mélange et dissout, et une fois la concentration de la solution résultante réglée de manière à se trouver dans l'intervalle de 3 % jusqu'à 10 %, on procède au refroidissement de la solution.

6. Procédé selon l'une quelconque des revendications 1 à 5, dans lequel la neutralisation est suivie d'un dessalage par dialyse.

7. Procédé selon la revendication 6, dans lequel on élimine 50 % ou plus du sel restant à la fin de de l'étape de neutralisation.

8. Procédé selon l'une quelconque des revendications 1 à 7, caractérisé en ce que l'ester méthylique d'α-L-aspartyl-L-phénylalanine obtenu après l'étape de cristallisation (c) est dissous à nouveau et la solution résultante est refroidie avec ou sans agitation.

9. Procédé selon la revendication 8, dans lequel on effectue le refroidissement sans agitation ou sous un écoulement forcé faible de sorte que tout ou partie du liquide soit obtenu sous forme de pseudo-phase solide de type sherbet.

10. Procédé selon l'une quelconque des revendications 1 à 8, dans lequel le sel d'addition acide de l'ester méthylique d'α-L-aspartyl-L-phénylalanine est le chlorhydrate de ce dernier.
